# EUROPEAN PATENT APPLICATION

(11) **EP 4 375 368 A1**
(43) Date of publication of application: **29.05.2024**
(21) Application number: 22855319.4
(22) Date of filing: 04.08.2022
(51) Int. Cl.: C12N 7/01, C07K 7/06, C07K 7/08, C07K 14/00, C12N 15/86, C12N 15/867, C12N 5/10, A61K 39/12, A61P 31/12

(54) **RECOMBINANT VIRUS CONTAINING DEGRON, PREPARATION METHOD THEREFOR, AND APPLICATION THEREOF**

(30) Priority: 12.08.2021 CN 202110923852
(71) Applicant: Shenzhen Institutes of Advanced Technology Chinese Academy of Sciences, Shenzhen, Guangdong 518055 (CN)
(72) Inventor: SI, Longlong, Shenzhen, Guangdong 518055 (CN)
(74) Representative: Grünecker Patent- und Rechtsanwälte PartG mbB
(86) International application number: PCT/CN2022/110258
(87) International publication number: WO 2023/016336

(57) **Abstract**

A recombinant virus containing a degron, a preparation method therefor, and an application thereof. At least one viral protein of the recombinant virus containing the degron contains at least one degron capable of being recognized by a protein degradation system of a host cell, wherein the degron comprises any one of or a combination of at least two of an amino acid sequence, a polypeptide, or a structural motif. Further provided are a nucleic acid molecule, a recombinant vector, a preparation method for the recombinant virus containing the degron, a preparation system for the recombinant virus containing the degron, a vaccine, an oncolytic virus, and a drug. The recombinant virus containing the degron can be recognized and degraded by the protein degradation system in the host cell, the replication capability is weakened or even removed, and after a corresponding vaccine, oncolytic virus or drug is prepared, a good effect and practical application value are achieved.

## Description

### TECHNICAL FIELD

The present application belongs to the field of biotechnology and, in particular, relates to a recombinant virus containing a degron, a preparation method therefor, and an application thereof.

### BACKGROUND

Virus infection seriously endangers human health and socio-economic development. A vaccine is one of the most effective means to prevent virus infection. Currently, the major virus vaccine types include inactivated vaccines, attenuated vaccines, subunit vaccines, viral vector vaccines, DNA vaccines, mRNA vaccines, and virus-like particles.

Although viral vaccine technology has developed significantly over the past decades, there are still some limitations. For example, the natural structure of an antigen is destroyed due to the virus inactivation processing, which reduces the immunogenicity of the viral vaccine; due to insufficiency of virus attenuation, the replication activity remains in the viral vaccine, which causes the vaccine to carry a safety hazard; or the preparation technology the virus vaccine cannot be extended to the preparation of other viral vaccines due to its complexity. Therefore, developing a new vaccine technology that is safe, efficient, simple, and universal by breaking through the conventional concept of virus vaccine design is an important direction for the development of ideal virus vaccines in the future and has important scientific and clinical values.

How to provide a method for preparing a virus vaccine with more safety, wider applicability, and stronger immunogenicity has become an urgent problem to be solved.

### SUMMARY

The present application provides a recombinant virus containing a degron, a preparation method therefor, and an application thereof. The recombinant virus prepared by inserting at least one degron into at least one protein coding gene of a virus is capable of being recognized by a protein degradation system of a host cell, the viral proteins are degraded, the replication capability of the virus is weakened or even removed, a resulting vaccine or oncolytic virus is safer, and the application prospect is promising.

In a first aspect, the present application provides a recombinant virus containing a degron, wherein at least one viral protein of the recombinant virus containing the degron contains at least one degron capable of being recognized by a protein degradation system of a host cell;
wherein the degron includes any one or a combination of at least two of an amino acid sequence, a polypeptide or a structural motif.

In the present application, at least one degron is inserted into at least one protein of a virus, the degron can be replicated as the viral genome is replicated and can be fused and expressed in the viral protein as the viral protein is translated, and a recombinant virus that is site-specific modified by a degron is obtained. In addition, the stable degradation of viral proteins in the host cell is achieved by using the protein degradation system in the host cell, the replication and other life activities of the virus in the host are artificially controlled, and the recombinant virus can be used to prepare replication-controllable viral vaccines or oncolytic viruses, which is of great value for application.

In the present application, degrons refer to various molecules capable of being specifically recognized by a protein degradation system and further mediating substrate protein degradation, such as specific amino acid sequences, polypeptides, structural motifs, and other molecules. Degrons are generally a type of short linear motifs with a specific sequence pattern and show strong evolutionary conservatism for amino acid residues that are critical for interaction.

Preferably, the protein degradation system includes any one or a combination of at least two of an ubiquitin-proteasome system, a lysosomal system, an organelle hydrolysis system, a membrane surface hydrolysis system or a Caspase protease system.

In the present application, the normal execution of viral protein functions is a prerequisite for all life activities of a virus such as replication in host cells. A variety of natural protein degradation systems exist in host cells and are essential for maintaining intracellular protein homeostasis and thus maintaining normal cell functions. The protein degradation system can specifically recognize degrons in substrate proteins. An important feature of degrons is that they can be transferred, and after these degrons are connected through genetic engineering, long-lived proteins become unstable. These protein degradation systems and their corresponding degrons provide a biological basis for the design of "life switches" that can regulate the stabilization and degradation of viral proteins in the host cells.

Preferably, the degron is located at any one or a combination of at least two of a C-terminus, N-terminus or an intermediate coding region of the viral protein.

In the present application, the protein degradation systems of animals and humans can recognize a variety of degrons, and therefore different types and numbers of degrons can be introduced at the N-terminus, C-terminus or other arbitrary sites of the viral protein. These different types and numbers of degrons can be combined in any way to provide assurance for the preparation of viral vaccines with different replication efficiencies and different attenuation degrees, which is essential for the production efficiency and immunogenicity of the viral vaccine.

Preferably, the degron includes any one of the amino acid sequences represented by SEQ ID No. 1 to SEQ ID No. 110:
SEQ ID No. 1: ALAPYIP;
SEQ ID No. 2: DRHDSGLDSM;
SEQ ID No. 3: SHGFPPEVEEQDDGTLPMSCAQESGMDRHPAACASARINV;
SEQ ID No. 4: FVNQHLCGSHLVEALYLVCGERGFFYTPKA;
SEQ ID No. 5: DSGXXS (wherein X represents any amino acid);
SEQ ID No. 6: DRHDSGXXSM (wherein X represents any amino acid);
SEQ ID No. 7: LGSWRHWRGQEG;
SEQ ID No. 8: SLYKKVVGTMAAG;
SEQ ID No. 9: MLSESRNFPAQA;
SEQ ID No. 10: ERAPTGRWGRRG;
SEQ ID No. 11: KKVGRARPCQRG;
SEQ ID No. 12: APRAPRQRSRDG;
SEQ ID No. 13: SWRLTGSGMKG;
SEQ ID No. 14: WRPGRRGPSSGG;
SEQ ID No. 15: LRGPSPPPMAGG;
SEQ ID No. 16: WRPGRRGPSSGG;
SEQ ID No. 17: MALAVRVVYCGA;
SEQ ID No. 18: KKNVEAIGLLGG;
SEQ ID No. 19: PPGPPLSSPRPR;
SEQ ID No. 20: TMNNEEDLLRSL;
SEQ ID No. 21: TMEPPGGRQKKR;
SEQ ID No. 22: QERGPTWDKNLR;
SEQ ID No. 23: GTMAVLRQRPGR;
SEQ ID No. 24: LCTRSWDVTPNR;
SEQ ID No. 25: ANQPAQCRKTRI;
SEQ ID No. 26: EEARLKYDKSRI;
SEQ ID No. 27: LNDGPKPGQSRF;
SEQ ID No. 28: TSLYKKVGMGRK;
SEQ ID No. 29: YKKVGTMRGRGL;
SEQ ID No. 30: VGTMAAGRAPGK;
SEQ ID No. 31: KKVGTMRGVGYP;
SEQ ID No. 32: EGPLWHPRICGS;
SEQ ID No. 33: EMALSPPRSWGQ;
SEQ ID No. 34: RSGLRRRRHRGE;
SEQ ID No. 35: VSGIMRRPWGMN;
SEQ ID No. 36: KRVLIRVTYCGL;
SEQ ID No. 37: MALAVRVVYCGA;
SEQ ID No. 38: PSSPVQTTPLSQAVATPSRSSAAAAAALDLSGRRG;
SEQ ID No. 39: HHGKGFFGSGHYTAYCYNTEGGACALLCGVGDTERG;
SEQ ID No. 40: VAEITKQLPPVVPVSKPGALRRSLSRSMSQEAQRG;
SEQ ID No. 41: KKRPPPGLDR;
SEQ ID No. 42: SVNSLLKELR;
SEQ ID No. 43: HEWVLREGEE;
SEQ ID No. 44: EYLLKMATEE;
SEQ ID No. 45: ELCKSYRRLQ;
SEQ ID No. 46: VALKFKPRKH;
SEQ ID No. 47: GLGCKVLRRH;
SEQ ID No. 48: RHCGRT;
SEQ ID No. 49: RSHGTL;
SEQ ID No. 50: RFRGLR;
SEQ ID No. 51: RNLGIR;
SEQ ID No. 52: RGRLTRNKGP;
SEQ ID No. 53: SLFRKRNKGK;
SEQ ID No. 54: RTAASGRRWG;
SEQ ID No. 55: GLLKRPCLRG;
SEQ ID No. 56: QRKLQRTSRG;
SEQ ID No. 57: PKSKVCQQRG;
SEQ ID No. 58: KRLLKGSQYG;
SEQ ID No. 59: PHKRLLKGSQYG;
SEQ ID No. 60: KESNDCSCGG;
SEQ ID No. 61: DCVCRGSTGG;
SEQ ID No. 62: VAPRSRDERG;
SEQ ID No. 63: AHQLQALRRG;
SEQ ID No. 64: LTGKG;
SEQ ID No. 65: YYCFFG;
SEQ ID No. 66: LEKGG;
SEQ ID No. 67: AAHKG;
SEQ ID No. 68: ALRRG;
SEQ ID No. 69: GGSGG;
SEQ ID No. 70: RDERG;
SEQ ID No. 71: SRVKG;
SEQ ID No. 72: PASGG;
SEQ ID No. 73: AIHGG;
SEQ ID No. 74: GAEAG;
SEQ ID No. 75: GSTGG;
SEQ ID No. 76: RGMGG;
SEQ ID No. 77: LVHAG;
SEQ ID No. 78: SLQTG;
SEQ ID No. 79: PVPGG;
SEQ ID No. 80: NYKSG;
SEQ ID No. 81: PRKQG;
SEQ ID No. 82: TPRGG;
SEQ ID No. 83: GCSGG;
SEQ ID No. 84: EAQRG;
SEQ ID No. 85: GKAWG;
SEQ ID No. 86: PAGGG;
SEQ ID No. 87: VVLYG;
SEQ ID No. 88: LTLKG;
SEQ ID No. 89: GFQSG;
SEQ ID No. 90: RVQWG;
SEQ ID No. 91: SRTEGQFGTTQSNGTFFNGASPGTPPAPSQHQQSLTSL;
SEQ ID No. 92: YRPIPFQPEGAGEGTDEDKSNRIGNNGLRLNDGNGNGQLAPSPTPQGTEAVRA;
SEQ ID No. 93: RKFSNNPQPNAISNGTSTSERPGEGATQGIVEEEVLQ;
SEQ ID No. 94: IRTESAEEAEMASVPNGSPSWHPGASHVVNGAAGHSN;
SEQ ID No. 95: WHEIEMESGEEAMEPANETGNTLNGSPSWHPSPSHVI;
SEQ ID No. 96: NRTDFAGEEDEMDGVLNGSPSWHAATSHIVNGATVHQ;
SEQ ID No. 97: NRTDTAAEAEMDSVLNGSPSWHPPAGHVVNGATVHRS;
SEQ ID No. 98: NRTDTAAEAEMDSVLNGSPSWHPPAGHVVNGAAVHRS;
SEQ ID No. 99: NRTEVLEGAEIPSTVNGSPSWHPADSRAVSGATGHSS;
SEQ ID No. 100: NRTEAPEGTELPSTVNGSPSWHPADSRAGSGATGHSS;
SEQ ID No. 101: NRTEAPEGTESERETPSAINGNPSWHLADSPAVNGAT;
SEQ ID No. 102: NRTEAPEGTESEVETPSAINGNPSWQLADSPAINGATGHSSSLDAREVIPMAAVKQQAL;
SEQ ID No. 103: NRTEAPEGTESDMETPSAINGNASWHLADSPAVNGAT;
SEQ ID No. 104: NRTEAPEGTGPEMETPSAINGNPAWHPADSPAVNGAT;
SEQ ID No. 105: NRTEAPEGTESDMETPSAINGNPSWHLADSPAVNGAT;
SEQ ID No. 106: NRTEAPEGTESEAETPSAINGNPSWHLADSPAVNGAT;
SEQ ID No. 107: NRTEAPEGTESEMETPSAINGNPSWHLADSPPANGAT;
SEQ ID No. 108: NRTEAPEGTDSEMETPSAINGNPAWHLADSPAVNGAT;
SEQ ID No. 109: NRTEAPEGTDSEMETPSAINGNPSWHLADSPVVNGAT; or
SEQ ID No. 110: NRTEAPEGTESEMETPSAINGNPSWHLADSPAVNGAT.

Preferably, the virus includes any one of influenza virus, human immunodeficiency virus (HIV), severe acute respiratory syndrome coronavirus 2 (SARS-CoV-2), hand-foot-and-mouth virus, coxsackievirus, hepatitis C virus (HCV), hepatitis B virus (HBV), hepatitis A virus, hepatitis delta virus, hepatitis E virus, Epstein-Barr virus (EB virus), human papillomavirus (HPV), herpes simplex virus (HSV), cytomegalovirus, varicella-zoster virus, vesicular stomatitis virus, respiratory syncytial virus (RSV), Dengue virus, Ebola virus, Marburg virus, Zika virus, severe acute respiratory syndrome coronavirus (SARS), Middle East respiratory syndrome virus, rotavirus, rabies virus, measles virus, adenovirus, poliovirus, echovirus, encephalitis B virus, tick-borne encephalitis virus, Hantaan virus, neotype enterovirus, rubella virus, mumps virus, parainfluenza virus, porcine reproductive and respiratory syndrome virus, hog cholera virus, foot-and-mouth disease virus, parvovirus, prion, smallpox virus, tobacco mosaic virus, phage, herpes virus, West Nile virus, norovirus, human bocaviruss or coronavirus, and preferably is any one of influenza virus, human immunodeficiency virus or severe acute respiratory syndrome coronavirus 2.

Preferably, any one or a combination of at least two of a PA protein, a PB1 protein, a PB2 protein, an NP protein, an HA protein, an NA protein, an M1 protein, an M2 protein, an NS1 protein or an NEP protein of the influenza virus contains at least one degron. For example, the PA protein and the PB2 protein of the influenza virus contain at least one degron; the PA protein and the PB1 protein of the influenza virus contain at least one degron; the PB2 protein and the PB1 protein of the influenza virus contain at least one degron; the PA protein, the PB2 protein, and the PB1 protein of the influenza virus contain at least one degron; the PA protein, the PB2 protein, the PB1 protein, and the M1 protein of the influenza virus contain at least one degron; the PA protein, the PB2 protein, the PB1 protein, the M1 protein, and the NP protein of the influenza virus contain at least one degron; the PB2 protein, the PB1 protein, and the M1 protein of the influenza virus contain at least one degron; the PA protein and the M1 protein of the influenza virus contain at least one degron; the PB1 protein and the M1 protein of the influenza virus contain at least one degron; the PB2 protein and the M1 protein of the influenza virus contain at least one degron; the PB2 protein, the PB1 protein, the M1 protein, and the NS1 protein of the influenza virus contain at least one degron; the PB2 protein, the PB1 protein, the M1 protein, and the NEP protein of the influenza virus contain at least one degron; and the NS1 protein and the NEP protein of the influenza virus contain at least one degron.

Preferably, any one or a combination of at least two of Gag polyprotein, pol polyprotein, gp160, HIV trans-activator of transcription (Tat), regulator of expression of virion proteins (Rev), negative factor (Nef), lentivirus protein R (Vpr), viral infectivity factor (Vif), virus protein U (Vpu), matrix protein (MA, p17), capsid protein (CA, p24), spacer peptide 1 (SP1, p2), nucleocapsid protein (NC, p7), spacer peptide 2 (SP2, p1), P6, reverse transcriptase (RT), Rnase H, integrase (IN), HIV protease (PR), gp120 or gp41 protein of HIV contains at least one degron.

Preferably, any one or a combination of at least two of spike protein (S protein), envelope glycoprotein (E glycoprotein), membrane glycoprotein (M glycoprotein), nucleocapsid protein (N protein), nonstructural protein 1 (nsp1), nonstructural protein 2, nonstructural protein 3, nonstructural protein 4, nonstructural protein 5, nonstructural protein 6, nonstructural protein 7, nonstructural protein 8, nonstructural protein 9, non-structural protein 10, nonstructural protein 11, nonstructural protein 12, nonstructural protein 13, nonstructural protein 14, nonstructural protein 15, nonstructural protein 16, 3a protein, 3b protein, 6 protein, 7a protein, 7b protein, 8a protein, 8b protein, 9b protein, 3C-like proteinase, leader protein, 2'-O-ribose methyltransferase, endoRNAse, 3'-to-5' exonuclease, helicase, RNA-dependent RNA polymerase, orfla polyprotein, ORF10 protein, ORF8 protein, ORF7a protein, ORF6 protein or ORF3a protein of SARS-CoV-2 contains at least one degron.

In the present application, the recombinant virus may be further modified by, for example, introducing some immunopotentiators into specific regions or specific amino acids of a virus protein, thereby obtaining a recombinant virus with improved performance and enhanced immunogenicity.

In a second aspect, the present application provides a nucleic acid encoding the recombinant virus containing the degron described in the first aspect.

In a third aspect, the present application provides a recombinant vector containing the nucleic acid molecule described in the second aspect.

Preferably, the recombinant vector expresses the recombinant virus containing a degron described in the first aspect.

In a fourth aspect, the present application provides a preparation method for the recombinant virus containing the degron described in the first aspect. The preparation method includes the following steps:
constructing a cell line with a protein degradation system defect;
introducing a nucleotide sequence encoding a degron into a coding gene of a viral protein; and
constructing the recombinant vector described in the third aspect, introducing the recombinant vector into the cell line with the protein degradation system defect, and packaging to obtain the recombinant virus containing the degron.

In the present application, since the protein degradation system is widely distributed in host cells, to avoid the reduction in production efficiency due to the degradation of the recombinant virus by the intracellular protein degradation system in the preparation process, the present application provides an artificially modified cell line with a protein degradation system defect. In the artificially modified cell line, the degrons are not recognized, and the viral proteins are not degraded by the protein degradation system and are retained so that the recombinant virus can be efficiently replicated and prepared on a large scale in the specific artificially modified cell line. In normal cells, the protein degradation system recognizes the degrons fused to the viral proteins and thus degrades the viral proteins, and the replication capability of the virus is weakened or even removed. Therefore, the recombinant virus is extremely safe.

The dependence of the recombinant virus on the specific virus production system allows for the large-scale preparation of recombinant viruses in the system. Since the protein degradation system exists in the normal cells of humans and animals, the degrons fused and expressed in the viral proteins can be recognized, and the viral proteins are thus degraded. Therefore, the recombinant virus prepared has a reduced replication capability or cannot be replicated and reproduced in animals and humans, thereby increasing the safety of the virus and making the recombinant virus a veritable live attenuated viral vaccine.

In the present application, the preparation principles of the recombinant virus are as follows: (1) the degron introduced into a specific site of a viral protein can be recognized by a protein degradation system in a normal host cell, thereby inducing degradation and inactivation of the relevant viral protein; (2) the degron introduced into a specific site of a viral protein cannot be recognized or inhibited in a specific viral production system, thereby avoiding or reducing the viral protein being degraded by a protein degradation system of a host cell; and (3) the degron introduced into a specific site of a viral protein is recognized by a protein degradation system in a normal host cell, the prepared virus can be recognized and degraded by the protein degradation system in the host cell of animals and humans, and thus the replication capability is weakened or even removed, thereby increasing the safety of the virus.

Preferably, the protein degradation system defect includes an ubiquitin-proteasome system defect, wherein the ubiquitin-proteasome system defect includes any one or a combination of at least two of E3 ligase knockout or knockdown or proteasome knockout or knockdown.

Preferably, the cell line includes a mammalian cell line, wherein the mammalian cell line includes any one of a CHO cell line, a Vero cell line, an MDCK cell line, an HEK293 T cell line, an MDCK cell line, an A549 cell line, a BHK cell line, a BHK-21BRS cell line, an Sp2/0 cell line, an HEK293 cell line, a 293F cell line, a HeLa cell line, a TZM-bl cell line, a Sup-T1 cell line, an MRC-5 cell line, a VMK cell line, an LLC-MK2 cell line, an HCT-8 cell line, an Huh-7 cell line or a Caco2 cell line, and preferably is any one of an HEK293 T cell line, an MDCK cell line or an A549 cell line.

In the present application, the use of the mammalian cell line further solves the disadvantage that a virus propagated using chicken embryos is prone to cause adverse reactions such as allergies in humans in the conventional method.

Preferably, the recombinant virus containing the degron is prepared by adding a protein degradation system inhibitor.

Preferably, the protein degradation system inhibitor includes a proteasome inhibitor, and the proteasome inhibitor includes any one or a combination of at least two of MG132, MG-341 or lactacystin.

Preferably, the preparation method further includes a step of detecting the replication capacity, security, and immunogenicity of the recombinant virus containing the degron in the cell line with the protein degradation system defect and in an unmodified cell line.

In the present application, whether the recombinant virus is successfully modified is determined by detecting the replication capacity of the recombinant virus containing the degron in the cell line with the protein degradation system defect and in an unmodified cell line. The recombinant virus that is replicated normally in the cell line with the protein degradation system defect and that has a weakened replication capability or cannot be replicated in the unmodified cell line is a successfully modified recombinant virus.

Preferably, the preparation method further includes a step of mass production.

As a preferred technical solution, the preparation method for the recombinant virus containing the degron provided by the present application includes the following steps:
(1) constructing a mammalian cell line with a protein degradation system defect:
   knocking out key parts of a protein degradation system in a cell line by gene editing technology;
(2) determining a virus protein into which a degron is introduced and a type, a quantity, and an introduction site of the degron, and introducing a nucleotide sequence encoding the degron into a coding gene of the virus protein to obtain a recombinant virus sequence;
(3) constructing a recombinant vector:
   connecting the recombinant virus sequence in step (2) to a plasmid to obtain the recombinant vector; and
(4) introducing the recombinant vector into the cell line with the protein degradation system defect, and packaging to obtain the recombinant virus containing the degron:
   co-transfecting the recombinant vector in step (3) and a virus rescue plasmid into the cell line with the protein degradation system defect by reverse genetic technology, adding a protein degradation system inhibitor, and packaging to obtain the recombinant virus containing the degron; and
   detecting a replication capacity, security, and immunogenicity of the recombinant virus containing the degron in the cell line with the protein degradation system defect and in an unmodified cell line, and performing mass production.

In step (2) of the present application, by performing bioinformatics analysis and protein structure prediction on an influenza virus, the protein structure of the virus obtained after the introduction of degron sequenced into individual proteins of the virus is predicted and analyzed to screen the viral proteins into which the degron has been introduced and to determine the type, the quantity, and the specific sites of the degron that have been introduced.

In step (4) of the present application, by using reverse genetic technology, any gene of an existing virus model may be replaced with a gene of another subtype or strain or an existing virus model may be replaced with another subtype or strain to prepare a recombinant virus of another subtype or strain. In this manner, the method may be applied to a virus of any subtype or strain, and the prepared virus is replicated on a large scale in a cell line with a protein degradation system defect and is recognized by a protein degradation system in normal host cells.

In the present application, for a recombinant virus that has been successfully modified, steps (2) to (4) may be repeated to achieve the introduction of a degron on all of a plurality of viral proteins of the recombinant virus or the introduction of a plurality of degrons on any of the viral proteins of the recombinant virus.

In addition, a polyvalent virus may also be prepared by using reverse genetic technology, and more importantly, the prepared mutant virulent virus and polyvalent virus are highly safe and effective.

In a fifth aspect, the present application provides a system for preparing the recombinant virus containing the degron described in the first aspect. The system includes a cell line with a protein degradation system defect, a recombinant vector, and a virus rescue plasmid.

In a sixth aspect, the present application provides an application of any one or a combination of at least two of the recombinant virus containing the degron described in the first aspect, the nucleic acid molecule described in the second aspect, the recombinant vector described in the third aspect, the method for preparing the recombinant virus containing the degron described in the fourth aspect or the system for preparing the recombinant virus containing the degron described in the fifth aspect in the preparation of a vaccine and/or a drug.

In a seventh aspect, the present application provides a vaccine containing the recombinant virus containing the degron described in the first aspect.

Preferably, the vaccine includes any one of a live attenuated vaccine, a replication-incompetent live vaccine, a replication-controllable live vaccine or an oncolytic virus vaccine.

Preferably, the vaccine further includes an adjuvant and an auxiliary material.

In an eighth aspect, the present application provides an oncolytic virus, and the oncolytic virus contains the recombinant virus containing the degron described in the first aspect.

In a ninth aspect, the present application provides a drug, and the drug contains the recombinant virus containing the degron described in the first aspect.

Preferably, the drug further includes any one or a combination of at least two of a pharmaceutically acceptable carrier, a diluent or an excipient.

Compared with the prior art, the present application has the following beneficial effects.
(1) Universality: Since viral replication can be controlled simply by introducing a degron into a viral protein, the technical scheme of the present application can modify all viruses and can be used in the preparation of all viral vaccines; a variety of protein degradation systems and the corresponding degrons can be selected, with fewer constraints and greater applicability.
(2) Simple operation: Only simple viral vector construction technology and virus packaging technology are required, and the technologies are mature and have a high success rate, promoting the preparation and promotion of related products.
(3) Good effect: Vaccines with different degrees of inactivation can be designed according to the technical scheme of the present application, which are safe and controllable; the prepared vaccine has excellent immunogenicity and has a broad application prospect.

### BRIEF DESCRIPTION OF DRAWINGS

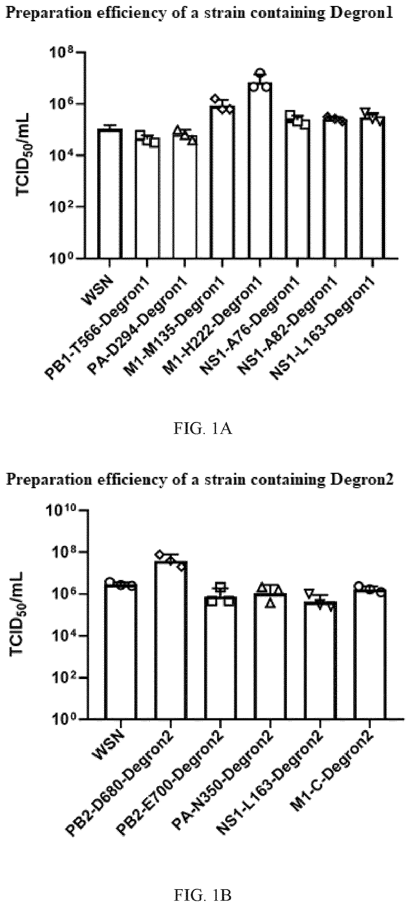

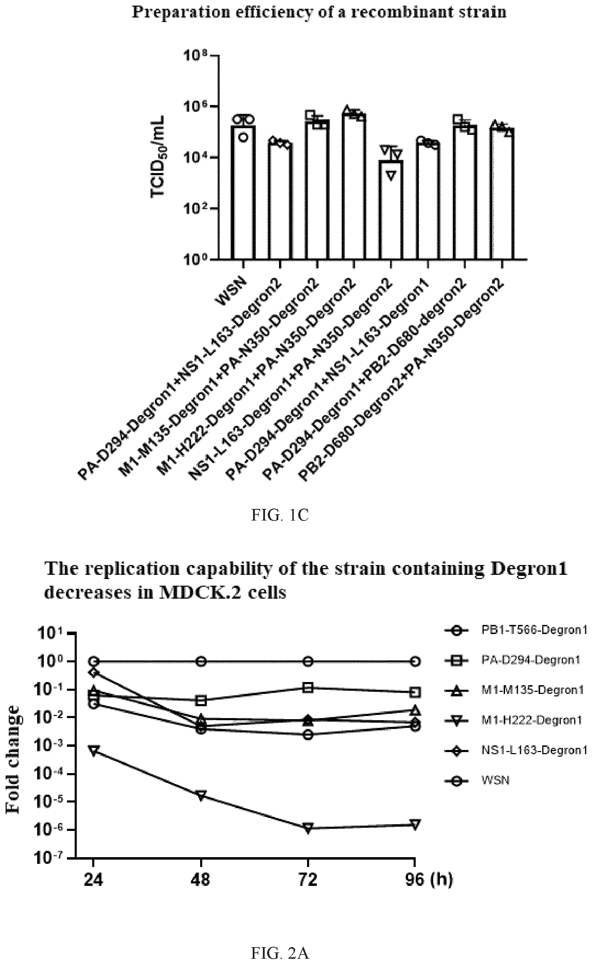

FIG. 1A shows the preparation efficiency of a strain containing Degron1 in Example 3; FIG. 1B shows the preparation efficiency of a strain containing Degron2 in Example 3; and FIG. 1C shows the preparation efficiency of a recombinant strain containing both Degron1 and Degron2 in Example 3.

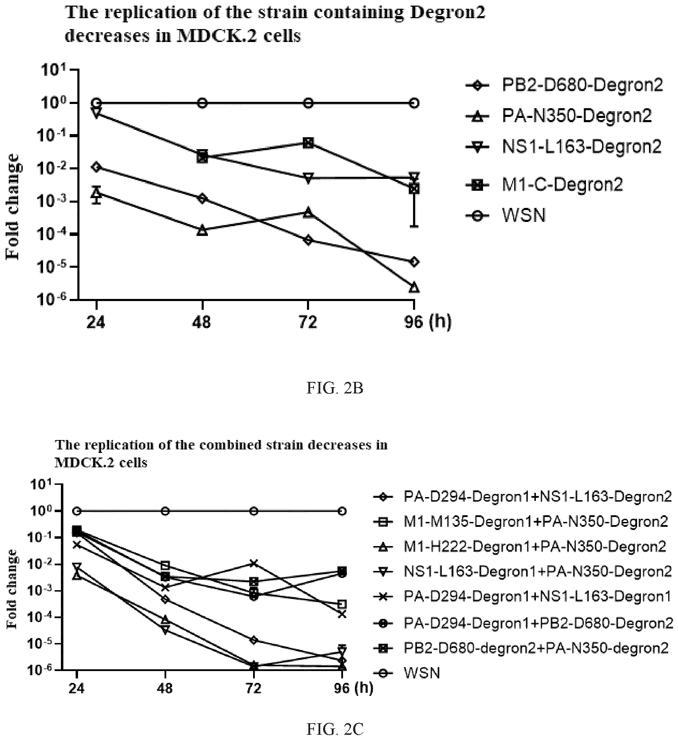

FIG. 2A shows the growth curve of the strain containing Degron1 in MDCK cells in Example 3; FIG. 2B shows the growth curve of the strain containing Degron2 in MDCK cells in Example 3; and FIG. 2C shows the growth curve of the recombinant strain containing multiple Degron1 and/or Degron2 in MDCK cells in Example 3.

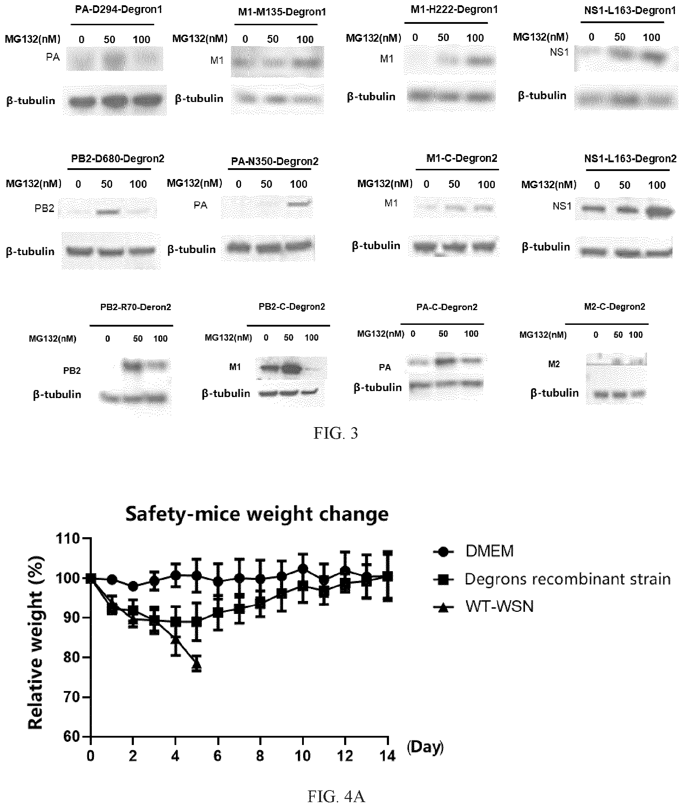

FIG. 3 shows viral protein expression levels detected by Western Blot in Example 4.

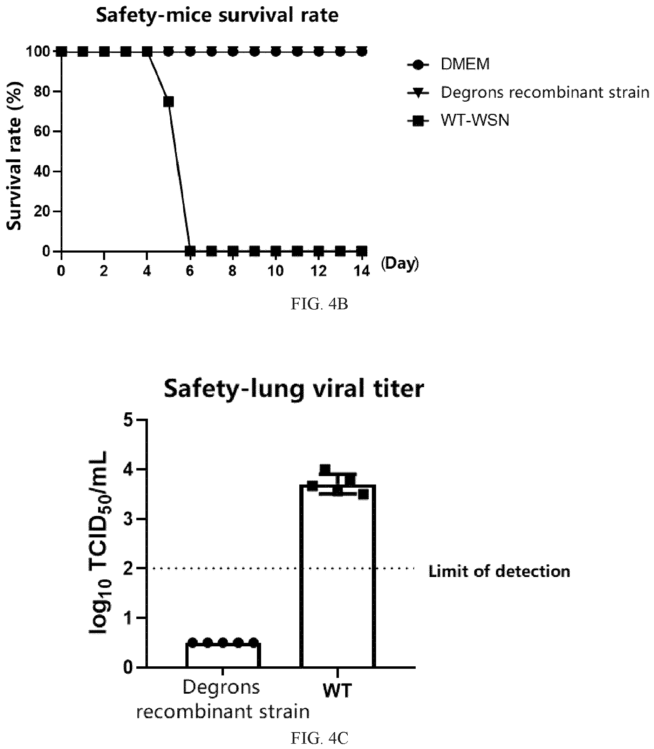

FIG. 4A shows the body weight change of mice inoculated with a recombinant influenza virus in Example 5; FIG. 4B shows the survival rate of mice inoculated with a recombinant influenza virus in Example 5; and FIG. 4C shows the viral titer in the lungs of mice inoculated with a recombinant influenza virus in Example 5.

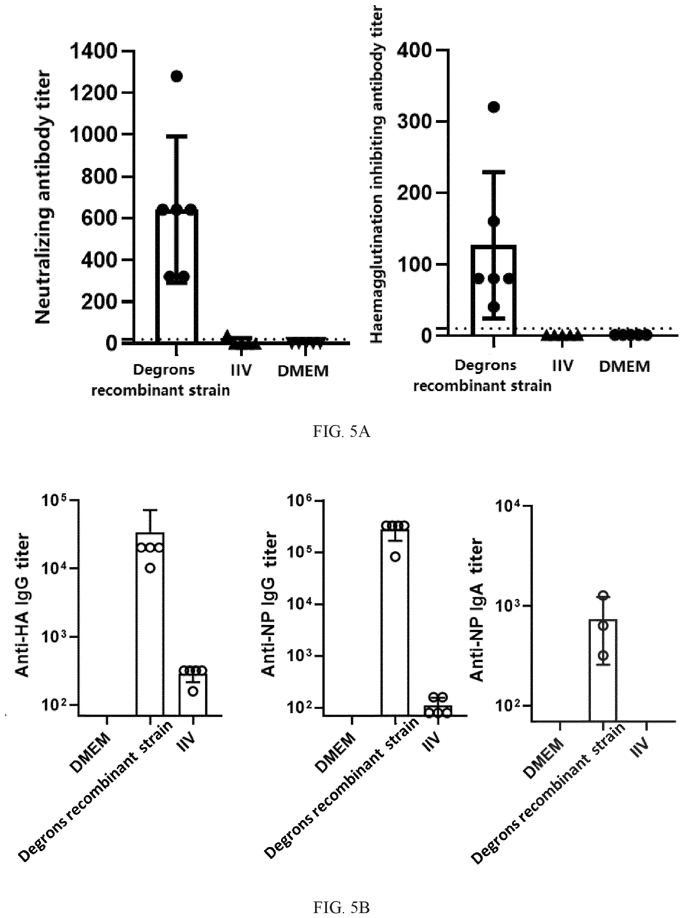

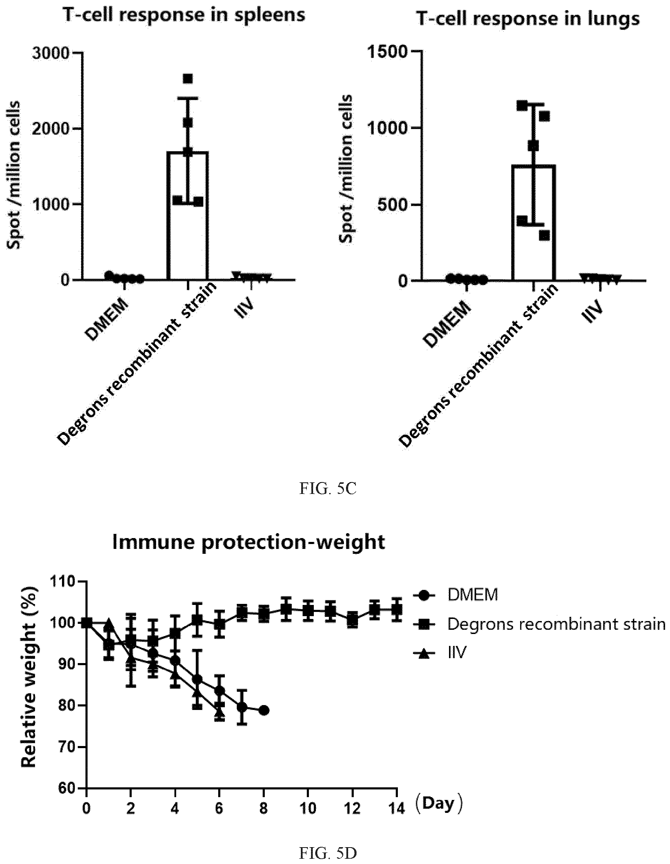

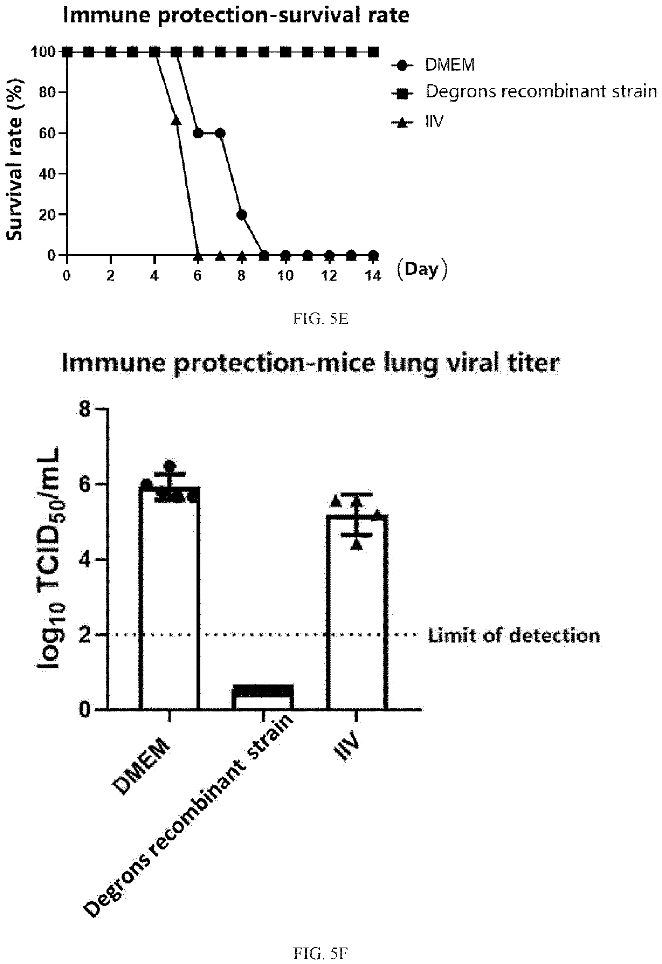

FIG. 5A shows the neutralizing antibody titer and haemagglutination inhibiting antibody titer after inoculation with a recombinant influenza virus in Example 6; FIG. 5B shows the anti-NP IgG, anti-HA IgG, and anti-NP IgA titers after inoculation with a recombinant influenza virus in Example 6; FIG. 5C shows the T-cell responses in the spleens and lungs after inoculation with a recombinant influenza virus in Example 6; FIG. 5D shows the body weight change of mice inoculated with a recombinant influenza virus after wild-type homologous influenza strain (WSN) attack in Example 6; FIG. 5E shows the survival rate of mice inoculated with a recombinant influenza virus after wild-type homologous influenza strain (WSN) attack in Example 6; and FIG. 5F shows the viral titer in the lungs of mice inoculated with a recombinant influenza virus after wild-type homologous influenza strain (WSN) attack for three days in Example 6.

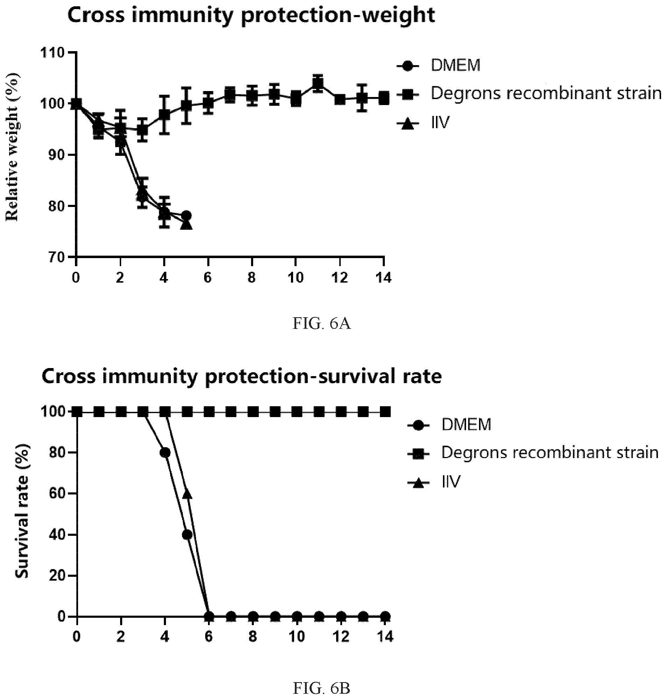

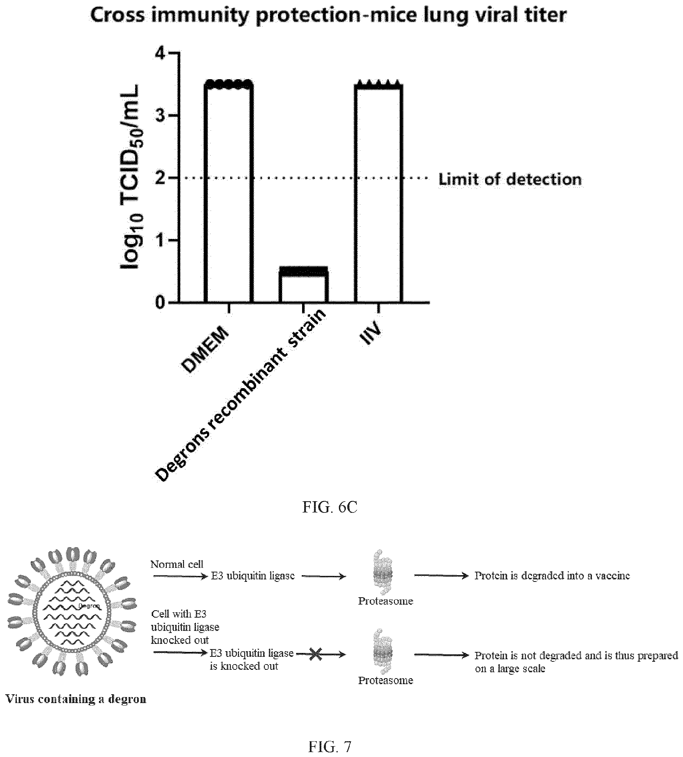

FIG. 6A shows the body weight change of mice inoculated with a recombinant influenza virus after wild-type heterologous influenza strain (H3N2) attack in Example 7; FIG. 6B shows the survival rate of mice inoculated with a recombinant influenza virus after wild-type heterologous influenza strain (H3N2) attack in Example 7; and FIG. 6C shows the viral titer in the lungs of mice inoculated with a recombinant influenza virus after wild-type heterologous influenza strain (H3N2) attack for three days in Example 7.
FIG. 7 shows the working principle of a virus containing a degron: in normal cells, the degron covalently linked to a viral protein induces E3 ubiquitin ligase to recognize the viral protein and ubiquitinates the viral protein, then the proteasome degrades the ubiquitinated viral protein, and finally, the virus is attenuated to become a vaccine; in cells with E3 ubiquitin ligase knocked out, the degron is unable to induce the viral protein to be ubiquitinated, then the viral protein will not be degraded by the proteasome and is retained, and finally, the virus is replicated as efficiently as a wild-type virus and prepared on a large scale.

### DETAILED DESCRIPTION

To further elaborate on the technical means adopted and effects achieved in the present application, the present application is further described below in conjunction with examples. It is to be understood that the specific examples set forth below are intended to explain the present application and are not to limit the present application.

Experiments without specific techniques or conditions specified in the examples are conducted according to techniques or conditions described in the literature in the art or according to product specifications. The reagents or instruments used herein without manufacturers specified are conventional products commercially available from proper channels.

### Materials:

pHH21, pCDNA3(neo), and pcAAGGS/MCS vectors, purchased from Beijing Zhongke Yubo Biotechnology Co., Ltd.

### Example 1

This example provides a recombinant vector expressing a recombinant influenza virus containing a degron, and the recombinant vector was prepared by the following method:

### (1) Construction of virus rescue plasmids

According to the genetic sequence of influenza virus A/WSN/1933 published by Pubmed:
https://www.ncbi.nlm.nih.gov/nuccore/?term=WSN+PB2,
https://www.ncbi.nlm.nih.gov/nuccore/?term=WSN+PB1,
https://www.ncbi.nlm.nih.gov/nuccore/?term=WSN+PA,
https://www.ncbi.nlm.nih.gov/nuccore/?term=WSN+HA,
https://www.ncbi.nlm.nih.gov/nuccore/?term=WSN+NA,
https://www.ncbi.nlm.nih.gov/nuccore/?term=WSN+NP,
https://www.ncbi.nlm.nih.gov/nuccore/?term=WSN+M, and
https://www.ncbi.nlm.nih.gov/nuccore/?term=WSN+NS.

The gene of each gene segment of the influenza virus was obtained by gene synthesis. The genes were ligated into pHH21, pCDNA3(neo), and pcAAGGS/MCS vectors to obtain wild-type influenza virus rescue plasmids, respectively. The names and compositions of the obtained plasmids are shown in Table 1.

**Table 1**

| Abbreviation | Plasmid name | Key gene | Enzyme site | Plasmid structure |
|---|---|---|---|---|
| Ben1 | PHH21 | PB2 | BsmBI | pPolI-WSN-PB2 |
| Ben2 | PHH21 | PB1 | BsmBI | pPolI-WSN-PB1 |
| Ben3 | PHH21 | PA | BsmBI | pPolI-W SN-PA |
| Ben4 | PHH21 | HA | BsmBI | pPolI-WSN-HA |
| Ben5 | PHH21 | NP | BsmBI | pPolI-WSN-NP |
| Ben6 | PHH21 | NA | BsmBI | pPolI-WSN-NA |
| Ben7 | PHH21 | M | BsmBI | pPolI-WSN-M |
| Ben8 | PHH21 | NS | BsmBI | pPolI-WSN-NS |
| Ben9 | pcDNA3(neo) | PB2 | EcoRI | pcDNA3(neo)-PB2 |
| Ben 10 | pcDNA3(neo) | PB1 | EcoRI | pcDNA3 (neo)-PB 1 |
| Ben 11 | pcDNA3(neo) | PA | EcoRI | pcDNA3(neo)-PA |
| Ben 13 | pcAGGS/MCS | NP | EcoRI | pcAGGS/MCS-NP |

### (2) Construction of recombinant vectors

The coding sequences of the degrons were introduced into the viral proteins and ligated to the plasmids to construct a series of recombinant plasmids. This work was supported by Beijing Tsingke Biotech Co., Ltd, and the mutant construction was verified to be successful by sequencing.

The naming principles of the recombinant vectors are as follows:
1. When the degron is introduced at the N-terminus of the viral protein, the constructed viral vector is named "viral protein name-N-degron name";
2. When the degron is introduced at the C-terminus of the viral protein, the constructed viral vector is named "viral protein name-C-degron name"; and
3. When the degron is introduced into the coding region of the viral protein, the constructed viral vector is named "viral protein name-name and amino acid number of the upstream amino acid adjacent to the introduction site-degron name".

The constructed recombinant plasmids are as follows:
PB2-N-Degron1, PB2-R70-Degron1, PB2-I176-Degron1, PB2-V457-Degron1, PB2-N510-Degron1, PB2-Y531-Degron1, PB2-A623-Degron1, PB2-D680-Degron1, PB2-E700-Degron1, PB2-C-Degron1, PB1-N-Degron1, PB1-D70-Degron1, PB1-D295-Degron1, PB1-R327-Degron1, PB1-R430-Degron1, PB1-F490-Degron1, PB1-T566 Degron1, PB1-N626-Degron1, PB1-G710-Degron1, PB1-C-Degron1, PA-N-Degron1, PA-D294-Degron1, PA-N350-Degron1, PA-E372-Degron1, PA-L425-Degron1, PA-H510-Degron1, PA-A553-Degron1, PA-E604-Degron1, PA-S624-Degron1, PA-C-Degron1, NP-N-Degron1, NP-G126-Degron1, NP-N247-Degron1, NP-R317-Degron1, NP-V353-Degron1, NP-A366-Degron1, NP-Q409-Degron1, NP-E465-Degron1, NP-M481-Degron1, NP-C-Degron1, M1-N-Degron1, M1-A33-Degron1, M1-V68-Degron1, M1-D89-Degron1, M1-R105-Degron1, M1-M135-Degron1, M1-Q164-Degron1, M1-H222-Degron1, M1-A239-Degron1, M1-C-Degron1, M2-C-Degron1, NEP-C-Degron1, NS1-N-Degron1, NS1-A76-Degron1, NS1-A82-Degron1, NS1-H101-Degron1, NS1-A122-Degron1, NS1-T151-Degron1, NS1-L163-Degron1, NS1-C-Degron1, HA-N-Degron1, HA-C-Degron1, NA-N-Degron1, NA-C-Degron1, PB2-N-Degron2, PB2-R70-Degron2, PB2-I176-Degron2, PB2-V457-Degron2, PB2-N510-Degron2, PB2-Y531-Degron2, PB2-A623-Degron2, PB2-D680-Degron2, PB2-E700-Degron2, PB2-C-Degron2, PB1-N-Degron2, PB1-D70-Degron2, PB1-D295-Degron2, PB1-R327-Degron2, PB1-R430-Degron2, PB1-F490-Degron2, PB1-T566 Degron2, PB1-N626-Degron2, PB1-G710-Degron2, PB1-C-Degron2, PA-N-Degron2, PA-D294-Degron2, PA-N350-Degron2, PA-E372-Degron2, PA-L425-Degron2, PA-H510-Degron2, PA-A553-Degron2, PA-E604-Degron2, PA-S624-Degron2, PA-C-Degron2, NP-N-Degron2, NP-G126-Degron2, NP-N247-Degron2, NP-R317-Degron2, NP-V353-Degron2, NP-A366-Degron2, NP-Q409-Degron2, NP-E465-Degron2, NP-M481-Degron2, NP-C-Degron2, M1-N-Degron2, M1-A33-Degron2, M1-V68-Degron2, M1-D89-Degron2, M1-R105-Degron2, M1-M135-Degron2, M1-Q164-Degron2, M1-H222-Degron2, M1-A239-Degron2, M1-C-Degron2, M2-C-Degron2, NEP-C-Degron2, NS1-N-Degron2, NS1-A76-Degron2, NS1-A82-Degron2, NS1-H101-Degron2, NS1-A122-Degron2, NS1-T151-Degron2, NS1-L163-Degron2, NS1-C-Degron2, HA-N-Degron2, HA-C-Degron2, NA-N-Degron2, and NA-C-Degron2.

The sequence of Degron1 is represented by SEQ ID No. 1, and the encoding nucleotide sequence is represented by SEQ ID No. 111; the sequence of Degron2 is represented by SEQ ID No. 2, and the encoding nucleotide sequence is represented by SEQ ID No. 112.
SEQ ID No. 111: GCATTGGCCCCCTACATTCCA;
SEQ ID No. 112: GATCGCCACGATTCAGGGCTCGATTCCATG.

### Example 2

This example provides a recombinant influenza virus containing a degron, and the recombinant influenza virus containing a degron was prepared by the following method:
(1) A mammalian cell line with a protein degradation system defect was constructed:
   The parts of a protein degradation system in the cell line were knocked out by using CRISPR/Cas9 knockout technology.
(2) The recombinant vector was introduced into the cell line with the protein degradation system defect, and packaged to obtain a recombinant virus containing a degron:
   the recombinant vector in Example 1 replaced the corresponding wild-type plasmid and co-transfected the mammalian cell line with the protein degradation system defect together with 11 other plasmids (for example, the recombinant vector PB2-N-Degron1 replaced plasmid Ben1 and co-transfected the mammalian cell line together with 11 other plasmids), the addition amount of each plasmid was 0.2 µg, and the transfected cells were incubated in a DMEM medium containing 0.5% FBS and 2 µg/mL TPCK-trypsin.

Four days after transfection, after the host cells were completely diseased or more than 90% diseased, the supernatant was collected, new cells with a protein degradation system defect were infected in a DMEM medium containing 0.5% FBS and 2 µg/mL TPCK-trypsin, a protein degradation system inhibitor was added, and four days after infection, after the host cells were completely diseased, the supernatant was collected.

The supernatant was centrifuged and filtered through a 0.4 µm filter membrane to remove cell debris, and the packaged product was assayed for dependence of the protein degradation system defect as well as for dependence of inactivation of the packaged product on the protein degradation system, and the mutants that maintained dependence of the protein degradation system defect were retained as the recombinant virus.

The prepared recombinant viruses were named in the same manner as the recombinant vectors.

### Example 3

The recombinant influenza viruses containing the degron were evaluated for preparation efficiency and cell-level safety (shown in Table 2 below).

**Table 2**

| Strain | TCID₅₀/mL | copy number/mL |
|---|---|---|
| PB1-T566 Degron1 | 10^4~10^5.8 | 10^8.84~10^11.68 |
| PA-D294-Degron 1 | 10^6.2~10^6.33 | 10^8.55~10^11.76 |
| PA-N3 50-Degron 1 | 10^4.67~10^6.67 | 10^9.32-10^11.94 |
| M 1-M 13 5-Degron 1 | 10^3.43~10^5.57 | 10^8.21~10^11.31 |
| M1-H222-Degron1 | 10^3.67~10^6 | 10^8.71~10^11.54 |
| NS1-A76-Degron1 | 10^4.67~10^6.2 | 10^9.25~10^11.63 |
| NS1-A82-Degron1 | 10^5~10^6 | 10^9.17~10^11.78 |
| NS 1-L 163-Degron 1 | 10^4.2~10^6.3 | 10^8.38~10^11.63 |
| PB2-N-Degron2 | 10^7.17 | 10^6.97~10^7.14 |
| PB2-R70-Degron2 | 10^5.41 | 10^7.33~10^7.96 |
| PB2-I176-Degron2 | 10^6.4 | 10^7.87 |
| PB2-V457-Degron2 | 10^6.3 | 10^8.3215 |
| PB2-D680-Degron2 | 10^6.2 | 10^7.32~10^7.89 |
| PB2-E700-Degron2 | 10^5.67 | 10^7.92~10^8.03 |
| PB2-C-Degron2 | 10^4.5 | 10^7.32~10^7.58 |
| PA-N350-Degron2 | 10^5.25 | 10^7.57~10^7.97 |
| PA-C-Degron2 | 10^5.54 | 10^7.35~10^8.43 |
| M2-C-Degron2 | / | 10^7.63~10^7.71 |
| NS1-N-Degron2 | 10^6.33 | 10^8.5 |
| NS1-A76-Degron2 | 10^4.88 | 10^8.03 |
| NS1-A82-Degron2 | 10^7.42 | 10^8.34 |
| NS 1-H101-Degron2 | NT | 10^7.87~10^8.41 |
| NS1-L163-Degron2 | 10^5.1 | 10^7.81 |

### (1) Preparation efficiency of the recombinant influenza viruses containing the degron

The recombinant influenza viruses containing the degron and a wild-type influenza virus infected cell lines with E3 ubiquitin ligase knocked out (MOI = 0.01), respectively, the cell supernatants were collected 48 hours after culture, and the viral titers were detected, respectively. The titers of the recombinant influenza viruses containing the degron were compared to the titer of the wild-type virus to determine the preparation efficiency of the recombinant influenza viruses containing the degron. In this example, strains containing Degron1, Degron2, and combinations of Degron1 and Degron2 respectively were used to characterize the preparation efficiency of the recombinant influenza viruses containing the degron. The results show that, as shown in the figures (FIG. 1), in HEK293T cells with VHL E3 ubiquitin ligase knocked out, the titers of all recombinant influenza strains containing Degron1 were comparable to the titer of the wild-type WSN influenza virus; in HEK293T cells with β-TrCPE3 ubiquitin ligase knocked out, the titers of all recombinant influenza strains containing Degron2 were comparable to the titer of the wild-type WSN influenza virus; the titers of all recombinant influenza strains containing multiple Degron1 and/or Degron2 were comparable to the titer of the wild-type WSN influenza virus. These results indicate that the recombinant influenza virus containing the degron has higher preparation efficiency.

### (2) Cell-level safety of the recombinant influenza viruses containing the degron

The growth curves of the recombinant influenza viruses containing the degron and the wild-type influenza virus in normal MDCK cells were examined to determine the safety of the strains. The criterion for the safety of the strain is as follows: if the replication capability of the strain is weakened or even removed (the viral titer was lower than the titer of the wild-type virus) in a normal MDCK cell line as compared to the wild-type virus, the strain is safe. In this example, strains containing Degron1, Degron2, and combinations of Degron1 and Degron2 respectively were used to characterize the safety of the recombinant influenza viruses containing the degron. The steps of the detection based on the growth curves are as follows.

The prepared recombinant influenza viruses containing the degron and the wild-type influenza virus infected normal MDCK cell lines in a ratio of MOI = 0.01, respectively, and at 24 h, 48 h, 72 h, and 96 h after infection, the virus titers in the cells were detected with qPCR to detect the replication capacity of the viruses in the cells. The results (in FIG. 2) show that the replication capability of the recombinant influenza virus containing the degron was weakened or even removed in the normal MDCK cell line as compared to the wild-type influenza virus. The results indicate that the prepared recombinant influenza virus containing the degron is safe at the cell level.

### Example 4

The mechanism of attenuation of the recombinant influenza virus containing the degron was verified.

The recombinant influenza viruses containing the degron and the wild-type influenza virus infected the normal MDCK cell line (MOI = 0.1), respectively, and proteasome inhibitors MG-132 of 50 nM and 100 nM were supplemented in the medium, respectively, and DMSO (at the same dilution ratio as the viruses) was used as a control. 48 h after infection, the cell samples were collected and detected for the viral protein expression level by Western Blot.

The results (FIG. 3) show that the recombinant influenza virus containing the degron could not be replicated on a large scale after infecting the normal MDCK cells, and thus fewer signals of virus protein M1 were detected; when the proteasome system of the cells was inhibited, the signal of the virus protein M1 increased, indicating that the replication capability of the virus increases after the proteasome system was inhibited. It is proved that the introduction of protein hydrolysistargeting molecules mediates the degradation of viral proteins by the proteasome of the cell, which in turn inhibits the replicative capacity of the virus; this is consistent with the design principle of the recombinant influenza virus containing the degron.

### Example 5

Evaluation of safety of the recombinant influenza viruses containing the degron at the animal level:
The safety of the recombinant influenza viruses containing the degron at the animal level was evaluated using C57BL/6J mice. With the recombinant strain containing both Degron1 and Degron2 (which was named the Degrons recombinant strain) as the representative, the virus safety evaluation was performed.

Specific steps of the safety evaluation are as follows.
(a) Thirty 7-week-old female C57BL/6J mice were divided into three groups, 10 mice per group.
(b) Each mouse in the first group was inoculated with DMEM by intranasal administration, each mouse in the second group was inoculated with 10⁵ TCID₅₀ Degrons recombinant strain by intranasal administration, and each mouse in the third group was inoculated with 10⁵ TCID₅₀ wild-type WSN influenza virus by intranasal administration.
(c) Three days after inoculation, the lung tissues of five mice in each group were taken, and the viral titers in the lung tissues were detected.
(d) The weight and death situation of the remaining five mice in each group were observed and monitored for 14 days.

The results (FIG. 4) show that the wild-type WSN influenza virus could be highly replicated in the lungs of mice and caused significant weight loss and death in mice; the recombinant influenza virus containing the degron has a titer below the limit of detection in the lungs of mice and does not cause weight loss or death in mice. Therefore, the safety of the recombinant influenza virus vaccine containing the degron is good.

### Example 6

Examination of immunogenicity and protectiveness of the recombinant influenza virus vaccine containing the degron at the animal level:
Immunogenicity and protectiveness of the recombinant influenza virus containing the degron at the animal level were evaluated. With an inactivated influenza vaccine (IIV) as a control (the inactivated influenza virus vaccine was prepared by the inventors using homologous influenza virus particles according to the method provided by *Chinese Pharmacopoeia*) and the recombinant strain containing both Degron1 and Degron2 (which was named the Degrons recombinant strain) as the representative, the immunogenicity and protectiveness of the recombinant influenza virus vaccine containing the degron were evaluated.

Specific steps of the immunogenicity and protectiveness examination are as follows.
(1) Sixty 7-week-old female C57BL/6J mice were divided into three groups, 20 mice per group.
(2) Each mouse in the first group was inoculated with DMEM by intranasal administration, each mouse in the second group was inoculated with 10⁵ TCID₅₀ Degrons recombinant strain by intranasal administration, and each mouse in the third group was inoculated with 10⁵ PFU inactivated influenza vaccine (IIV) by intranasal administration.
(3) One week after inoculation, the lung tissues and spleens of five mice in each group were taken, and the immune responses of the T cells in the lung tissues and spleens were detected.
(4) Three weeks after inoculation, the blood of five mice in each group was collected for haemagglutination inhibition (HI) test, neutralizing (NT) antibody test, and ELISA assay, respectively, and the antibody immune response in the blood was detected.
(5) Three weeks after inoculation, the mice in each group were inoculated with 2 × 10⁵ TCID₅₀ wild-type WSN influenza virus by intranasal administration.
(6) Three days after inoculation with the wild-type WSN influenza virus, the lung tissues of five mice in each group were taken, and the viral titers in the lung tissues were detected.
(7) The weight and death situation of the remaining five mice in each group were observed and monitored for 14 days.

The results (FIG. 5) show that the Degrons recombinant strain could induce high levels of haemagglutination inhibition antibody titer, neutralizing antibody titer, anti-NP IgG, anti-HA IgG, and anti-NP IgA within animals; the levels of haemagglutination inhibition antibody, neutralizing antibody, anti-NP IgG, anti-HA IgG, and anti-NP IgA induced by the Degrons recombinant strain were significantly higher than the levels of antibodies induced by the inactivated influenza vaccine; the vaccination with the Degrons recombinant strain could provide complete immune protection, including inhibition of the viral titer in the lungs of the mice below the limit of detection to enable all the mice to survive with their body weights unchanged; the vaccination with the inactivated vaccine only provided a limited immune protective effect, for example, the levels of wild-type influenza virus replication were still high in the lungs of mice and part of mice survived with their body weights significantly lost. These data indicate that the Degrons recombinant strain vaccine provides significantly better protection than the inactivated influenza vaccine. Therefore, the recombinant influenza virus vaccine containing the degron has more excellent immunogenicity and protection effects.

### Example 7

Examination of the cross immunity protection effect of the recombinant influenza virus vaccine containing the degron at the animal level:
Specific steps are as follows.
(1) ten 7-week-old female C57BL/6J mice were divided into three groups, 10 mice per group.
(2) Each mouse in the first group was inoculated with DMEM by intranasal administration, each mouse in the second group was inoculated with 10⁵ TCID₅₀ Degrons recombinant strain by intranasal administration, and each mouse in the third group was inoculated with 10⁵ PFU inactivated influenza vaccine (IIV) by intranasal administration.
(3) Three weeks after inoculation, the mice in each group were inoculated with 103 TCID₅₀ wild-type influenza virus H3N2 by intranasal administration.
(4) Three days after inoculation with the wild-type influenza virus H3N2, the lung tissues of five mice in each group were taken, and the viral titers in the lung tissues were detected.
(5) The weight and death situation of the remaining five mice in each group were observed and monitored for 14 days.

The results (in FIG. 6) show that the vaccination with the Degrons recombinant strain could provide cross immunity protection, including inhibition of the viral titer in the lungs of the mice below the limit of detection to enable all the mice to survive with their body weights unchanged; the vaccination with the inactivated vaccine could not provide cross immunity protection, for example, the levels of influenza virus H3N2 in the lungs of the mice were comparable to the levels in unvaccinated mice, and all of the mice died with their body weights significantly lost. These data indicate that the Degrons recombinant strain vaccine provides significantly better cross immunity protection than the inactivated influenza vaccine. Therefore, the recombinant influenza virus vaccine containing the degron has more excellent cross-immunogenicity and protection effects.

In summary, in the present application, with the introduction of a degron into a viral protein, the prepared recombinant virus can be recognized and degraded by a protein degradation system in a host cell, which weakens or even removes the replication capability of the recombinant virus; the recombinant virus can be replicated in cells with a protein degradation system defect, thereby achieving the large-scale production thereof; the corresponding vaccine or drug prepared from the recombinant virus has excellent safety and immunogenicity and has a broad application prospect; the method for preparing recombinant virus is extremely adaptable and simple to operate, which promotes the use and promotion of the product.

The applicant has stated that although the detailed method of the present application is described through the examples described above, the present application is not limited to the detailed method described above, which means that the implementation of the present application does not necessarily depend on the detailed method described above. It is to be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

## Claims

1. A recombinant virus containing a degron, wherein at least one viral protein of the recombinant virus containing the degron contains at least one degron capable of being recognized by a protein degradation system of a host cell;
wherein the degron comprises any one or a combination of at least two of an amino acid sequence, a polypeptide or a structural motif.

2. The recombinant virus containing the degron according to claim 1, wherein the protein degradation system comprises any one or a combination of at least two of a ubiquitin-proteasome system, a lysosomal system, an organelle hydrolysis system, a membrane surface hydrolysis system or a Caspase protease system.

3. The recombinant virus containing the degron according to claim 1 or 2, wherein the degron is located at any one or a combination of at least two of a C-terminus, N-terminus or an intermediate coding region of the viral protein.

4. The recombinant virus containing the degron according to any one of claims 1 to 3, wherein the degron comprises any one of amino acid sequences represented by SEQ ID No. 1 to SEQ ID No. 110.

5. The recombinant virus containing the degron according to any one of claims 1 to 4, wherein the virus comprises any one of influenza virus, human immunodeficiency virus, severe acute respiratory syndrome coronavirus 2, hand-foot-and-mouth virus, coxsackievirus, hepatitis C virus, hepatitis B virus, hepatitis A virus, hepatitis delta virus, hepatitis E virus, Epstein-Barr virus, human papillomavirus, herpes simplex virus, cytomegalovirus, varicella-zoster virus, vesicular stomatitis virus, respiratory syncytial virus, Dengue virus, Ebola virus, Marburg virus, Zika virus, severe acute respiratory syndrome coronavirus, Middle East respiratory syndrome virus, rotavirus, rabies virus, measles virus, adenovirus, poliovirus, echovirus, encephalitis B virus, tick-borne encephalitis virus, Hantaan virus, neotype enterovirus, rubella virus, mumps virus, parainfluenza virus, porcine reproductive and respiratory syndrome virus, hog cholera virus, foot-and-mouth disease virus, parvovirus, prion, smallpox virus, tobacco mosaic virus, phage, herpes virus, West Nile virus, norovirus, human bocaviruss or coronavirus, and preferably is any one of influenza virus, human immunodeficiency virus or severe acute respiratory syndrome coronavirus 2.

6. A nucleic acid molecule encoding the recombinant virus containing the degron according to any one of claims 1 to 5.

7. A recombinant vector containing the nucleic acid molecule according to claim 6;
preferably, the recombinant vector expresses the recombinant virus containing the degron according to any one of claims 1 to 5.

8. A preparation method for the recombinant virus containing the degron according to any one of claims 1 to 5, comprising:
constructing a cell line with a protein degradation system defect;
introducing a nucleotide sequence encoding a degron into a coding gene of a viral protein; and
constructing the recombinant vector according to claim 7, introducing the recombinant vector into the cell line with the protein degradation system defect, and packaging to obtain the recombinant virus containing the degron;
preferably, the protein degradation system defect comprises a ubiquitin-proteasome system defect, and the ubiquitin-proteasome system defect comprises any one or a combination of at least two of E3 ligase knockout or knockdown or proteasome knockout or knockdown;
preferably, the cell line comprises a mammalian cell line, wherein the mammalian cell line comprises any one of a CHO cell line, a Vero cell line, an MDCK cell line, an HEK293 T cell line, an MDCK cell line, an A549 cell line, a BHK cell line, a BHK-21/BRS cell line, an Sp2/0 cell line, an HEK293 cell line, a 293F cell line, a HeLa cell line, a TZM-bl cell line, a Sup-T1 cell line, an MRC-5 cell line, a VMK cell line, an LLC-MK2 cell line, an HCT-8 cell line, an Huh-7 cell line or a Caco2 cell line, and preferably is any one of an HEK293 T cell line, an MDCK cell line or an A549 cell line;
preferably, the recombinant virus containing the degron is prepared by adding a protein degradation system inhibitor;
preferably, the protein degradation system inhibitor comprises a proteasome inhibitor, and the proteasome inhibitor comprises any one or a combination of at least two of MG132, MG-341 or lactacystin.

9. The preparation method according to claim 8, wherein the preparation method further comprises a step of detecting a replication capacity, security, and immunogenicity of the recombinant virus containing the degron in the cell line with the protein degradation system defect and in an unmodified cell line;
preferably, the preparation method further comprises a step of mass production;
preferably, the preparation method comprises:
(1) constructing a mammalian cell line with a protein degradation system defect:
knocking out key parts of a protein degradation system in a cell line by gene editing technology;
(2) determining a virus protein into which a degron is introduced and a type, a quantity, and an introduction site of the degron, and introducing a nucleotide sequence encoding the degron into a coding gene of the virus protein to obtain a recombinant virus sequence;
(3) constructing a recombinant vector:
connecting the recombinant virus sequence in step (2) to a plasmid to obtain the recombinant vector; and
(4) introducing the recombinant vector into the cell line with the protein degradation system defect, and packaging to obtain the recombinant virus containing the degron:
co-transfecting the recombinant vector in step (3) and a virus rescue plasmid into the cell line with the protein degradation system defect by reverse genetic technology, adding a protein degradation system inhibitor, and packaging to obtain the recombinant virus containing the degron; and
detecting a replication capacity, security, and immunogenicity of the recombinant virus containing the degron in the cell line with the protein degradation system defect and in an unmodified cell line, and performing mass production.

10. A system for preparing the recombinant virus containing the degron according to any one of claims 1 to 5, comprising a cell line with a protein degradation system defect, a recombinant vector, and a virus rescue plasmid.

11. An application of any one or a combination of at least two of the recombinant virus containing the degron according to any one of claims 1 to 5, the nucleic acid molecule according to claim 6, the recombinant vector according to claim 7, the preparation method for the recombinant virus containing the degron according to claim 8 or 9 or the system for preparing the recombinant virus containing the degron according to claim 10 in the preparation of a vaccine, an oncolytic virus and/or a drug.

12. A vaccine containing the recombinant virus containing the degron according to any one of claims 1 to 5;
preferably, the vaccine comprises any one of a live attenuated vaccine, a replication-incompetent live vaccine, a replication-controllable live vaccine or an oncolytic virus vaccine;
preferably, the vaccine further comprises an adjuvant and an auxiliary material.

13. An oncolytic virus containing the recombinant virus containing the degron according to any one of claims 1 to 5.

14. A drug containing the recombinant virus containing the degron according to any one of claims 1 to 5;
preferably, the drug further comprises any one or a combination of at least two of a pharmaceutically acceptable carrier, a diluent or an excipient.
